**Europäisches Patentamt**

**European Patent Office**

**Office européen des brevets**

(11) Publication number: **0 121 952**
**A2**

(12) **EUROPEAN PATENT APPLICATION**

(21) Application number: **84200204.0**

(22) Date of filing: **15.02.84**

(51) Int. Cl.³: **A 61 F 13/18, A 61 L 15/00**

(30) Priority: **15.02.83 NL 8300564**

(43) Date of publication of application: **17.10.84**
**Bulletin 84/42**

(84) Designated Contracting States: **BE DE FR GB NL**

(71) Applicant: **Tevic International B.V., Bergstraat 23-25, NL-7161 EE Neede (IT)**

(72) Inventor: **Verbeeten, Wilhelmus Franciscus Johannes, No. 7, Gorseveldweg, Hengevelde (NL)**

(74) Representative: **Cammel, Willem Frans et al, OCTROOIBUREAU ARNOLD & SIEDSMA No. 1, Sweelinckplein, NL-2517 GK The Hague (NL)**

(54) **Fluid absorbing product.**

(57) A moisture absorbing product with a moisture absorbing core and a moisture permeable envelope covering said core, which core contains at least flocks of a synthetic foam having a porous, internally hydrophilic, open network structure, and starch particles mixed with said flocks.

Eventually the core contains in addition one ore two different pH buffering compounds.

EP 0 121 952 A2

ACTORUM AG

# FLUID ABSORBING PRODUCT

The invention relates to a moisture absorbing product comprising a moisture absorbing core and a moisture permeable envelope covering said core.

Such a moisture absorbing product is generally known, for example, in the form of an incontinency undersheet, a diaper, a plaster, a tampon or a sanitary towel. In these known products the core contains as a moisture absorbing material, for example, cellulose and/or starch derivatives.

Further research has shown that the absorption capacity can be appreciably improved when in accordance with the invention the core contains at least flakes of a synthetic foam having a porous, internally hydrophilic, open network structure and starch particles mixed with the flakes.

As a result of the internally hydrophilic nature of the network structure the moisture absorbed by the core will not only penetrate into the network structure, but also form a gelatinous mass by the strongly expanding starch particles absorbing the moisture. This gelatinous mass extending into the network structure prevents the moisture adhering to the hydrophilic inner surface of the network structure from flowing out of said structure since the channels in the network structure are substantially completely closed by the gelatinous mass so that the moisture collected in the network structure can no longer flow away.

Research has shown that it is important for the synthetic foam to be available in the form of flocks mixed with the starch particles so that the open network structure is readily accessible over a short distance for the starch particles mixed with the flocks.

It is furthermore found, that the flocks and the starch particles mixed therewith cohere so little to one another, that such a moisture absorbing core very readily assumes the shapes of the body both in the dry and in the wet state. In this way phenomena such as bedsores and skin irritations ca be avoided.

Preferably viscose is used as a synthetic foam, because by nature viscose has a hydrophilic internal surface and satisfactory elastic properties, whilst being relatively cheap.

When the starch particles contain carboxymethylated starch the swelling power of the starch particles by moisture absorption is raised and hence the closure of channels in the network structure is improved.

When the core contains, in addition, at least one pH buffering compound, it is ensured that swelling of the starch particles is delayed. After the absorption of moisture by the core the swelling starch particles have a smaller external size for a longer time, so that more starch particles can penetrate into the network structure, which results in a higher moisture absorbing and retaining capacity. When preferably the pH buffering compound contains phosphate groups, a buffering effect is obtained in the pH range of 5.6 to 6.5. Moreover, the compound containing phosphate groups is hygroscopic, so that it is in addition substantially avoided that the starch particles absorb moisture from the atmosphere, which moisture absorption would have a negative effect on the swelling properties of the starch particles.

- 3 -

If it is desired, when using a moisture absorbing product, to avoid so-called ammonia burns, it is preferred for the core to contain furthermore a second pH buffering compound, which buffers at a lower pH than the first pH buffering compound, the second pH buffering compound preferably being citric acid or a salt thereof.

Since the highly improved moisture absorption of the product embodying the invention is based on the occlusion of moisture inside the network structure with the aid of the gelatinous mass of starch particles, a smaller amount of starch particles will be sufficient with respect to the amount of visco flocks. A products having a satisfying effect comprises, for example, 50 to 70 gs of viscose flocks and 10 to 20 gs of starch particles.

A moisture absorbing product in accordance with the invention may be formed by a moisture permeable envelope in the form of a fibre skin of polypropene provided with a moisture arresting substrate. The envelope surrounds the core containing at least flocks of, for example, viscose and starch particles mixed with the viscose flocks. The viscose to be processed to flocks having a porous, by nature internally hydrophilic, open network structure can be obtained from waste material of the production of, for example, synthetic sponges. This waste material is processed to viscose flocks with the aid of milling machines internally comprising coarse to very fine knives. The size of the flocks varies from about 3 to 10 mms.

Prior to filling the envelope the viscose foam flocks can be mixed with the starch particles.

For example, potato starch may be used, which is carboxymethylated for enhancing the hydrophilic nature, the substitution degree being equal, for example, to 0.63 mol/mol.

The following Tabel 1 enumerates a few properties of the carboxymethylated starch to which a pH buffering compound, for example, sodium dihydrogen phosphate is added.

TABEL 1: Properties of the carboxymethylated starch
containing a phosphate buffer.

| | |
|---|---|
| moisture content | 4 to 8% |
| pH (3% in distilled water) | 5 to 8 |
| substitution degree | 0.60 to 0.65 mol/mol |
| ash content (heated at 700°C) | ca. 35% |
| free salts content in ash | 20% |
| sodium glycolate | 6.5% |
| chloride (Cl⁻) | 11% |
| Phosphate ($P_2O_5$) | 1.1% |
| Sulphate ($SO_4^{2-}$) | 1.2% |

With the aid of the Enslin test procedure the water retention is assessed for various moisture absorbing materials, in which the material was soaked with water and subsequently the soaked material was subjected to a centrifugal process.

The following Tabel 2 gives the test results:

| Material | Water retention (g) |
|---|---|
| viscose flocks (100 gs) | 30 to 40 |
| starch particles (100 gs) | 300 to 350 |
| cellulose (100 gs) | 80 to 150 |
| Viscose flocks (110 gs)/starch part. (100 gs) | 900 to 1000 |
| Viscose flocks (61 gs)/starch part. (15 to 18 gs) | 480 to 520 |

This clearly illustrates the synergic effect when the viscose flocks and the starch particles are mixed. It is furthermore apparent that a considerable smaller amount by weight of starch particles may be used because there is only need for an amount of starch particles sufficient for blocking the channels in the network structure of the viscose floam flocks.

If the pH of the moisture absorbed in the core has to be further reduced, for example, to a pH lower than 5.5, citric acid or a salt thereof may be added as a second pH buffering compound. Citric acid is a reasonable cheap substance having a buffer effect in the pH range described above and tolerating well the other required constituents of the moisture absorbing core. Owing to this addition pH reduction the ammonia contained in the absorbed fluid is converted into an ammonium compound so that ammonia burns are avoided. For example, 0.1 to 0.2% of citric acid is added on the basis of the weight of the starch particles. It is necessary to take care that the pH value should not drop to pH 1 to 2, since at such a pH the absorption capacity of the used starch disappears due to the internal attack on the cross-link of the starch.

- 1 -

CLAIMS

1. A moisture absorbing product comprising a moisture absorbing core and a moisture permeable envelope covering said core, characterized in that the core contains at least flocks of a synthetic foam having a porous, internally hydrophilic, open network structure, and starch particles mixed with said flocks.

2. A product as claimed in claim 1, characterized in that the synthetic foam is viscose.

3. A product as claimed in anyone of the preceding claims, characterized in that the starch particles contain carboxymethylated starch.

4. A product as claimed in anyone of the preceding claims, characterized in that the core contains in addition at least one pH buffering compound.

5. A product as claimed in Claim 4, characterized in that the pH buffering compound contains phosphate groups.

6. A product as claimed in claim 4 or 5, characterized in that the core furthermore contains a second pH buffering compound, which buffers at a lower pH than the first pH buffering compound.

7. A product as claimed in claim 6, characterized in that the second pH buffering compound is citric acid or a salt thereof.

8. A product as claimed in anyone of claims 2 to 7, characterized in that the product contains 50 to 70 gs of viscose flocks and 10 to 20 gas of starch particles.